# EUROPEAN PATENT SPECIFICATION

(11) **EP 2 860 176 B1**
(45) Date of publication and mention of the grant of the patent: **27.09.2017**
(21) Application number: 12878273.7
(22) Date of filing: 08.06.2012
(51) Int. Cl.: C07D 271/107, C07D 271/06, C07D 249/06, A61K 31/4192, A61K 31/4245, A61P 37/06, A61P 29/00, A61P 19/02, C07D 401/04, A61K 31/4439, A61K 31/454

(54) **HETEROCYCLIC GROUP CONTAINED AMINO-METHANOL DERIVATIVE, AND SALT, SYNTHETIC METHOD AND USE THEREOF**
IN EINER HETEROCYCLISCHEN GRUPPE ENTHALTENES AMINOMETHANOLDERIVAT UND SALZ, SYNTHESEVERFAHREN SOWIE VERWENDUNG
DÉRIVÉ D'AMINOMÉTHANOL COMPRIS DANS UN GROUPE HÉTÉROCYCLIQUE ET SON SEL, SON PROCÉDÉ DE SYNTHÈSE ET SON UTILISATION

(30) Priority: 07.06.2012 CN 201210186211
(43) Date of publication of application: 15.04.2015
(73) Proprietor: Beijing Foreland Biopharma Co., Ltd., Beijing 100176 (CN)
(72) Inventor: ZHANG, Xingmin, Beijing 100176 (CN); WANG, Ensi, Beijing 100176 (CN); GUO, Jing, Beijing 100176 (CN); NIU, Shengxiu, Beijing 100176 (CN); DAI, Zhuolin, Beijing 100176 (CN); ZHENG, Nan, Beijing 100176 (CN); JI, Liping, Beijing 100176 (CN); WANG, Zhenfang, Beijing 100176 (CN); LIANG, Tie, Beijing 100176 (CN)
(74) Representative: Inspicos P/S
(86) International application number: PCT/CN2012/076642
(87) International publication number: WO 2013/181840

(56) References cited:
- WO-A1-2010/078711
- CN-A- 102 250 035
- CN-A- 102 250 035
- SAMBASIVARAO KOTHA ET AL: "The Diels-Alder Approach for the Synthesis of Tetralin-Based [alpha]-Amino Acid Derivatives and their Modification by the Suzuki-Miyaura Cross-Coupling Reaction", SYNTHESIS, no. 4, 1 January 2004 (2004-01-01), pages 558-567, XP055212936, ISSN: 0039-7881, DOI: 10.1055/s-2004-815987

## Description

### Technical Field

The invention belongs to medical field, particularly relates to the treatment of organ transplant rejections and immune mediated inflammatory diseases, such as multiple sclerosis, systemic lupus erythematosus and rheumatoid arthritis.

### Background

The immune system is a self-defensive structure mainly consisting of lymphatic organs (thymus glands, lymph nodes, spleen, tonsils), lymphatic tissues within other organs, lymphocytes throughout the body, antigen presenting cell, and the like. The immune system also includes other leucocytes in blood, and plasma cells and mast cells in connective tissues. The key components of the immune system are lymphocytes, which endue the immune system with the capability of recognition and memory. The lymphocytes travel throughout the body via blood and lymph, migrating from one lymphatic organ or tissue to another lymphatic organ or tissue, and connecting the lymphatic organs or tissues scattered throughout the body to form a functional entirety. T cells and B cells are the most important immunocytes in human bodies. The normal functioning of each component of the immune system provides the guarantee for the relative stability of the body immune functions, and any deficiencies or hyperactions of the component would cause damage to the body.

The components of immune system reach the whole body widely and complicatedly, particularly with the continuous production, circulation, and regeneration of the immune cells and immune molecules. The immune system possesses a great recognizability, which can precisely detect a foreign substance and distinguish it from human's own healthy tissue in order to maintain body's relative stability. Simultaneously, the immune system can accept, transfer, enlarge, depot, and memorize the related immune information, and provide positive or negative responses and regulate the responsibility to the immune information. However, the malfunctions of the immune system are disadvantageous to human body: human's abnormal recognizability easily results in allergy phenomenon, or causes iterative infections conversely; the abnormal stabilizing ability may induce the immune system to give responses to self-cells, which gives rise to autoimmune diseases.

Immunosuppressive agent is a type of new medicine category, which developed from the foundation of the research on neoplasm-chemotherapy, organ transplantation, immunopathology, and clinical immunology, etc. It possesses immunosuppressive effects which inhibits abnormal immune responses, and is generally used in the therapy of organ transplant rejection and autoimmune diseases.

Cyclophosphamide (CTX) was firstly applied to clinical practice, which was discovered to be hydroxylated by hepatocyte microsomes to generate the alkylated metabolite behaving potent and lasting immune functions. It is utilized as an immunosuppressive agent in the treatment of nephritic syndrome, systemic lupus eythematosus, and rheumatoid arthritis and so on by means of killing the immune cells and influencing every phase of the immunologic processes. But it is restricted because of the relatively obvious side effects.

Glucocorticoid is the most commonly used immunosuppressive agent in clinic nowadays, which can inhibit body's immune responses by inhibiting the phagocytic functions of macrophages, reducing the production of an auto-antibody against an auto-antigen. It is generally used for treating acute inflammation, allergy, organ transplant rejection, and some autoimmune diseases, etc.

Azathioprine is an immunosuppressive agent, which inhibits T cells and B cells. Azathioprine has been an effective drug for preventing organ transplant rejection for years, and is also used for various autoimmune diseases.

Cyclosporine A (CsA) is a cytokine synthesis inhibitor, which inhibits gene transcription of T cells cytokine, interrupts T cells production, and interferes activation of T cells. It belongs to an inhibitor of T cells activation at early phase, which interrupts T cells activations at phase G0/G1 (G0, G1 and S are different phases of a cell cycle). Since 1980s, CsA has been successively applied to various organ and tissue transplantations, and achieved abroad successes, which opened a new era for organ transplantations.

FK506 is another kind of binder subsequent to the development of CsA, which can prevent various transplant rejections, especially is useful in liver transplantation. FK506 has immunosuppresive effect 10∼100 times stronger than CsA, and has lower acute or chronic rejection rate, lower infection rate, lower dosage of hormone, and less adverse reaction than CsA. FK506 can reverse acute rejection and is expected to replace CsA to be the first choice as an immunosuppressive agent after an organ transplant.

Rapamycin (RPM), firstly used for anti-transplant rejection, can effectively prevent rejection reaction. Rapamycin is able to decrease the acute rejection rate in combination with other drugs. It can specifically inhibit the phosphorylation and activity of protein kinase to inhibit cytokine induced protein and DNA synthesis. It is a late stage T cells and B cells activation inhibitor. RPM, as a novel immunosuppressant, not only can inhibit the immune cells, but also can hinder the proliferation and transmigration of vascular smooth muscle to reduce rejection reaction.

Mycophenolate mofetil, approved by American FDA, was applied in clinical rapidly. It has potent curative effects and a high selective effect on proliferative lymphocytes, as well as can prevent the formation of antibody through direct inhibition of B cells proliferation.

Due to the restrictions of selectivity and specificity, the above mentioned immunosuppressive agents will inevitably damage immune defense capacity of the patients when receiving the treatment, resulting in the descent anti-infection ability of patients, the increasing risk of malignant lesions, the injury of hematopoietic system, immune system, liver, kidney and gastrointestinal function, neural and endocrine function disorder, and inducing some allergic reactions, etc. For instance, cyclophosphamide may cause hair loss, and induce hemorrhagic cystitis, such as frequent micturition, urodynia, hematuria, and proteinuria; glucocorticoids may aggravate or induce infection, induce gastric ulcer, combined with hemorrhage and perforation, provoke metabolic disorders, raise blood pressure, blood glucose, and blood fat, cause osteoporosis, and evoke adverse reaction of insomnia by exciting central nervous system; azathioprine can cause cholestasis and hepatocellular damage; MTX induce canal damage symptoms, such as oral cavity ulcer, bloody stool, and so on, and can cause teratism and stillbirth; cyclosporin is toxic to kidney, liver and nervous system, and can lead to high blood pressure, secondary infection and onset of tumor; FK506 also has renal toxicity, even more than CsA in neurotoxicity, and causes damages on the islet β2 cells that induces diabetes; rapamycin can elicit leukopenia, thrombocytopenia and hyperlipidemia; mycophenolate mofetil can cause vomiting, diarrhea and other gastrointestinal symptoms, leukopenia, sepsis and high blood uric acid, hyperkalemia, myalgia or sleepiness, etc.

Sphingosine-1-phosphate enzyme receptor antagonist FTY720 and sphingosine (a kind of endogenous hemolytic lipid) have some structural similarities. Sphingosine is phosphorylated to form sphingosine-1-phosphatek enzyme, a homologous series ligands of its receptor family, induced by sphingosine enzyme. Activation of the receptor leads to the following physiological activities: cells differentiation, growth and survival, and regulations of cytoskeletal recombinant that can change adhesion and morphology of cells. In the normal immune responses, the proliferation of T lymphocytes and B lymphocytes is taken place in lymph nodes. They down-regulate the sphingosine-1- phosphate receptor expression when they are in the lymph nodes. Once their activation and proliferation are completed, they will up-regulate the number of sphingosine-1-phosphate receptor on cell surface, which allowed them to leave the lymph nodes. Lymphocyte sphingosine-1-phosphate enzyme receptors bind to their ligands resulting in the down-regulation of sphingosine-1-phosphate enzyme, and thus losing the function of separating from the lymph nodes. To the end, lymphocytes will adhere to the lymph nodes (1, 2, 3). Traditional immunosuppressive agents such as cyclosporine have the action mechanism of inhibiting activation of T lymphocytes and B lymphocytes. In contrast, sphingosine-1-phosphate enzyme receptor antagonist achieves the purpose by means of limiting lymphocytes within the lymph system other than impairing the immune responses by lymphocytes inactivation. Sphingosine-1-phosphate enzyme receptor antagonist can be used for the treatment of various transplant rejections and immune inflammatory diseases.

Sphingosine-1-phosphate enzyme receptor antagonist FTY720 was successfully developed by Novartis (Novartis), and conducted clinical trials on multiple sclerosis and transplant patients in America and Europe (4, 5), and has gotten through a phase II clinical trial. However, FTY720 acts on not only sphingosine-1-phosphate enzyme receptor-1 (S1P1), but sphingosine-1-phosphate enzyme receptor-3 (S1P3), therefore, it can cause side effects such as bradycardia (6).

CN 102 250 035 A discloses heterocyclic group containing amino-methanol derivatives.

WO 2010/078711 A and Sambasivaro Kotaha et al., Syntesis, No. 4, 2004, pages 558-567, disclose 1,2,3,4-tetrahydronaphthalenyl compounds.

### Summary

The present invention provides a heterocyclic group contained amino-methanol derivative of Formula I which is 7-[5-(3, 4-diethoxyphenyl)-2-[1,3,4]-oxdiazolyl]-2-amino-1,2,3,4-tetrahydro-naphthalen-2-yl-methanol, and a physiologically acceptable salt thereof.

As described above, except for special annotations, the following terms in the entire disclosure should be appreciated as follows:

"Alkyl" refers to an aliphatic hydrocarbon, straight chain or branched, comprising approximately 1 to 20 carbon atoms. The preferred alkyl contains about 1 to 8 carbon atoms, more preferably about 1 to 6 carbon atoms. In a particular embodiment, it is represented by C1-C3 if the alkyl contains 1 to 3 carbon atoms. Branched alkane is composed of one or more lower alkyl groups, such as methyl, ethyl or propyl, linked to a straight chain alkane. "Lower alkyl" refers to straight or branched chain having 1 to 6 carbon atoms.

"Aryl" refers to an aromatic monocyclic or polycyclic system, consisting of about 6 to 14 carbon atoms, generally 6 to 10 carbon atoms.

"Cycloalkyl" refers to a non-aromatic monocyclic or polycyclic system containing 3 to 10 carbon atoms, preferably 5 to 10 carbon atoms, more preferably 5 to 7 carbon atoms. The non-limiting examples of a suitable monocyclic cycloalkyl include: cyclopropyl, cyclopentyl, cyclohexyl, cycloheptane, etc. The non-limiting examples of a suitable polycyclic cycloalkyl include: decahydronaphthalene, norbornane, adamantane and the like.

"Halogen" refers to fluorine, chlorine, bromine, or iodine. The preferred are fluorine, chlorine and bromine.

"Acyl" means H-C(O)-, -C(O)- linked to alkyl or cycloalkyl as defined above. The core structure is connected by carbonyl. The preferred are acyls containing lower alkyl groups. A suitable acyl group includes formyl, acetyl or propionic acid.

"Alkoxy" means an alkyl-O- group, herein the alkyl as previously described. A suitable non-limiting example of alkoxy includes methoxy, ethoxy, n-propoxy, isopropyl and n-butoxy. The core structure is connected by etheroxy.

The term "replaced" or "substituted" means in a specific atom, one or more hydrogens are replaced by specific groups. If the normal valence of the specific atom do not exceed in the existing circumstances, then the replacement results in a stable compound.

The substituents and/or variables are allowed to combine only if the combination results in stable compounds. The "stable compounds" or "stable structure" means a compound is capable of fully and effectively being isolated to be a available purity degree in the reaction mixture, and becomes an effective ingredient of medication.

The term "replaced selectively" or "substituted selectively" means selective replacement can be taken by using specified groups, free radicals or other groups.

The term "purification", "pure form" or "in the form of isolation and purification" refers to the physical state of a compound isolated after a synthesis process (e.g. from a reaction mixture), crude drug, or a combination. Therefore, for a compound, the term "purification", "in a purified form" or "in the form of separation and purification" refers to the physical state of a compound is in the state after purification process or after the processing described herein with known skills(such as chromatographic separation, recrystallization, etc.). According to the standard requirements, in a sufficient purity, the analysis technology described herein or a famous high-level skill is also characteristic.

It is also worth noting that in the specification, routes, examples and tables of the present invention, any carbon atoms and heteroatoms that do not satisfy the valence principle are assumed to contain a sufficient number of hydrogen atoms to satisfy the valence principle.

When a functional group of a compound is called "protected", it means that the functional group is in the modified form to prevent the unwanted side reactions occurring in the reaction. Suitable protective groups will be verified by the general operation technology of organic experiment and methods mentioned in the standard textbooks (e.g. T. W. Greene et al, Protective Groups in organic Synthesis (1991), Wiley, New York.).

As used herein the word "composition", the purpose of it is to obtain a result directly or indirectly from combination of particular components in a specific quantity and any products combined from particular components in a specific quantity.

Herein prodrugs and solvates of the compounds in this invention are also carefully considered. Discussions concerning about prodrugs are provided in Pro-drugs as Novel Delivery Systems (1987) Vol. 14 of the A.C.S. Symposium Series, Bioreversible Carriers in Drug Design, (1987) Edward B. Roche, ed., American Pharmaceutical Association, written by T. Higuchi and V. Stella, and in the Pergamon Press. The word "prodrug(s)" means a compound (e.g. a drug precursor) is transformed in vivo into a compound of Formula (I), or pharmaceutical salt, hydrate or solvate thereof. This transformation may be taken place by multiple mechanisms (e.g. metabolism or chemical process), such as hydrolysis in the blood. Discussions about the application of prodrugs are provided in "Prodrugs as Novel Delivery Systems," Vol. 14 of the A.C.S. Symposium Series, Bioreversible Carriers in Drug Design, ed. Edward B. Roche, American Pharmaceutical Association, written by T. Higuchi and W. Stella, and in Pergamon Press, 1987.

For example, there is a hydroxyl group in a compound of Formula (I), then a prodrug thereof can be obtained from replacing the hydrogen atom in hydroxyl group by another substituent group. For instance, the substituent group can be (C1-C6) alkyl acyloxy methyl, 1-((C1-C6) alkyl acyloxy) ethyl, 1-methyl-1-((C1-C6) alkyl acyloxy) ethyl, (C1-C6) alkoxycarbonyl-O-methyl, N-(C1-C6) alkoxycarbonyl amino methyl, -succinyl, (C1-C6) alkanoyl, -amino (C1-C4) alkyl, -aryl acetyl and -aminoacyl, or -aminoacyl- -aminoacyl, wherein each aminoacyl is independently choosing from natural L-amino acids, P(O)(OH)2, -P(O)(O(C1-C6)alkyl)2 or glycosyl (the most fundamental reason is the removal of a hydroxyl existing in the form of hemiacetal in carbohydrate), etc.

According to an amino group existing in a compound of Formula (I), the prodrug of the compound can be obtained from replacing the hydrogen atoms in the amino group by other substituent groups. For instance, these substituent groups can be R-carbonyl, RO-carbonyl, NRR'-carbonyl (R and R' are independent), (C1-C10) alkyl, (C3-C7) cycloalkyl, phenyl, or R-carbonyl (-amino acyl is the natural existence or natural-amino acyl), -C(OH)C(O)OY1, wherein Y1 is H, (C1-C6) alkyl or phenyl, -C(OY2)Y3 wherein Y2 represents (C1-C4) alkyl and Y3 represents (C1-C6) alkyl, carbonyl (C1-C6) alkyl, amino (C1-C4) alkyl, or one-N- or di-N, N-(C1-C6) alkylamino alkyl, -C(Y4)Y5 wherein Y4 represents hydrogen or methyl and Y5 represents one-N- or di-N, N-(C1-C6) morpholino alkylamino, piperidine-1-yl or pyrrolidine-1-yl, etc.

One or more compounds in the present invention may exist in the forms of non-solvates, and solvates just similar as the solvates formed from the pharmaceutically acceptable solvents such as water and ethanol. Therefore, the invention herein includes solvates and non-solvates of the compound of Formula (I). "Solvate(s)" refer to the physical aggregates of a compound of the present invention and one or more solvent molecules, which include ions in different degrees and covalent bonds, such as hydrogen bonds. It has been confirmed that the solvates can be separated, for example, when the crystal lattice of the crystal was mixed in one or more solvent molecules. "Solvate(s)" contain two parts, the solvent phase and the separable solvate. There are quantities of solvate examples, including ethanol solvate, methanol solvate, etc. "Hydrate(s)" is a kind of solvates whose solvent molecules are water (H₂O) molecules.

The compound of the present invention can be arbitrarily prepared into solvates. The preparation methods of the solvates are well known. For example, it is described in M. Caira et al, J. Pharmaceutical Sci., 93 (3), 601-611 (2004), the method for preparing antifungal fluconazole solvates prepared by ethyl acetate and water. It is also described a similar preparation method of solvates and hydrates in E. C. van Tonder et al, AAPS PharmSciTech., 5 (1), article 12 (2004); and A. L. Bingham et al, Chem. Commun., 603-604 (2001). A typical, non-limiting preparation process is that at a temperature higher than normal temperature, dissolve the compound of the present invention into the required amount of the ideal solvent (organic solvent or water or mixed solvent of the above two), lower the temperature, lay and crystallize, and then separate out the crystal by using the standard methods. By I. R. spectroscopy analysis technology, it can be confirmed that the existence of the solvent (water) in the solvates (hydrate).

"Effective amount" or "therapeutically effective amount" refers to the amount of the compound of the present invention or a composition exhibits a significant effect in inhibiting the said diseases, and thus produces necessary therapeutic, improving, inhibiting or preventing effects.

The salt of a compound of Formula I also belongs to the present invention. Except specific indications, its salt can be understood according to a compound of Formula I. The salt in the invention refers to the acid salt formed by organic acid/inorganic acid, and the basic salt formed by organic alkali/inorganic alkali. In addition, when the basic functional group of a compound of Formula I is a pyridine or imidazole (but not limited to pyridine or imidazole), and acidic functional group is a carboxylic acid (but not limited to carboxylic acid), a zwitterion (inner salt) will be formed, which is also included in the salt of the invention.

Although other salts are also useful, it is preferred to choose pharmaceutically acceptable salts (e.g., non-toxic, physiologically acceptable). The salt of a compound represented by Formula I is formed by the compound and the same amount of acid/base by reaction in medium for the precipitation of the salt, or by cryodesiccation in aqueous medium.

Exemplary acid salts include: acetic acid salts, ascorbate salts, benzoate, benzene sulfonates, hydrogen sulfates, borates, butyrates, citrates, camphorates, camphorsulfonates, fumarates, hydrochlorides, hydrobromides, hydroiodides, lactates, maleates, mesylates, napsylates, nitrates, oxalates, phosphates, propionates, salicylates, succinates, sulfates, tartrates, thiocyanates, tosylates, etc. Some other acids are commonly acknowledged to be suitable for producing the pharmaceutically valuable salts for low-level drug compounds, and have been discussed, for example, in P. Stahl, Camille G et al. Handbook of Pharmaceutical Salts: properties, selection and use. (2002) Zurich: Wiley-VCH; S. Berge, et al. Journal of Pharmaceutical Sciences, (1977) 66(1) 1-19; P. Gould, World Journal of Pharmaceutical Sciences (1986) 33 201-217; Anderson, et al., The Practice of Medicinal Chemistry (1966), Academic Press, New York; Orange Book (Food and Drug Administration, Washington, D.C. Columbia website). In addition, the publications are all incorporated herein by references.

Typical basic salts include ammonium salts, alkali metal salts, such as sodium salts, lithium salts and potassium salts; alkaline-earth metal salts, such as calcium salts and magnesium salts; salts formed by organic bases (such as organic amines), for example, dicyclohexyl amine, tertiary butyl amines; as well as salts formed by amino acids, such as arginine, or lysine and the like. The lower nitrogen containing species can be divided into four parts by medium: lower alkyl halide (e.g. methyl, ethyl, or butyl chloride, bromide and iodide); dialkyl sulfates (e.g. dimethyl, diethyl and dibutyl sulfates); long chain halides (e.g. decyl, lauryl and octadecyl chlorides, bromides and iodides); aralkyl halides (e.g. benzyl and phenethyl bromides).

All the acid salts and basic salts are intended to be pharmaceutically acceptable salts within the scope, and all of the acid and basic salts for the purpose of invention are identified as free form equivalent to the corresponding compounds.

In the present disclosure, pharmaceutically acceptable esters include following groups: (1) carboxylic esters after esterification of hydroxyl groups, wherein carbonyl-free moieties of the esters are selected from the following: straight or branched alkyl, (e.g. acetyl, propyl, tertbutyl or n-butyl), alkoxyalkyl (e.g. methoxymethyl), aralkyl (e.g. benzyl), aryloxy alkyl (e.g. phenoxy methyl), aryl (e.g. phenyl selectively substituted by halogen, C1-4 alkyl or C1-4 alkoxy or amino); (2) sulfonic acid esters, such as alkyl or aralkyl sulfonyl (e.g. mesyl) (3) amino acid esters (e.g. L-valine acyl or L-isoleucine acyl); (4) phosphoric acid esters, for example, monophosphate, diphosphate or triphosphate; (5) phosphoric acid esters can be further esterified, such as by C1-20 alcohols or the reactive derivatives thereof, or by 2,3-di(C6-24) acyl glycerol.

The compound of Formula I contains asymmetric or chiral centers, hence have various stereoisomers. All stereoisomers as well as the mixtures thereof, including the racemic mixture, are parts of the present invention. For example, if a compound of Formula I contains a double bond or a fused ring, either cis or trans isomer, or mixtures thereof, are within the scope of the invention.

A diastereomeric mixture can be separated into various individual diastereomers, on the basis of the different physical chemical properties by means of known measures. For example, enantiomers can be separated through reaction with suitable optical active substances (such as chiral alcohols or Mosher's Morse acyl chloride) into diastereomers which are separated then and transformed (such as hydrolysis) into the various corresponding individual isomers. The compound of the Formula I which are probably atropisomers (such as substituted aryl) are also parts of the invention. Enantiomers can also be separated by using chiral chromatographic column.

The invention also includes isotope labeled compounds. In fact, it is common that one or more atoms are replaced by an atom different in atomic mass or mass number in nature. Isotopes included in the compound of the present invention include H, C, N, O, P, F, S, such as ²H, ³H, ¹³C, ¹⁴C, ¹⁵N, ¹⁸O, ¹⁷O, ³¹P, ³²P, ³⁵S, ¹⁸F and ³⁶S.

It is very useful for the isotope labeled (such as ³H and ¹⁴C) compound of Formula I in the substances and/or tissues distribution detections. Isotope tritium (³H) and ¹⁴C (¹⁴C) are widely applied since easily prepared and detected. In addition, compounds substituted by deuterium (²H) displayed advantageous therapeutic effects and better metabolic stability (such as increasing half life period *in vivo*, or reducing desire dose), hence they are useful in some cases. The synthesis process of the isotope labeled compound of Formula 1 refers to the published documents and/or the examples herein.

The compound of Formula I is:
Example 20, 7-[5-(3,4-diethoxyphenyl)-2-[1,3,4]-oxdiazolyl]-2-amino-1,2,3,4-tetrahydro-naphthalen-2-yl-methanol.

As described in the present invention, the salts of the compounds, i.e., the derivatives of 2-amino-1,2,3,4-tetrahydronaphthyl-2-methanol containing various substituents, are the salts of hydrochloric acid.

The illustrative methods for preparing the derivative of 2-amino-1,2,3,4-tetrahydronaphthyl-2-methanol containing various substituents include the reactions and synthetic steps as shown below:

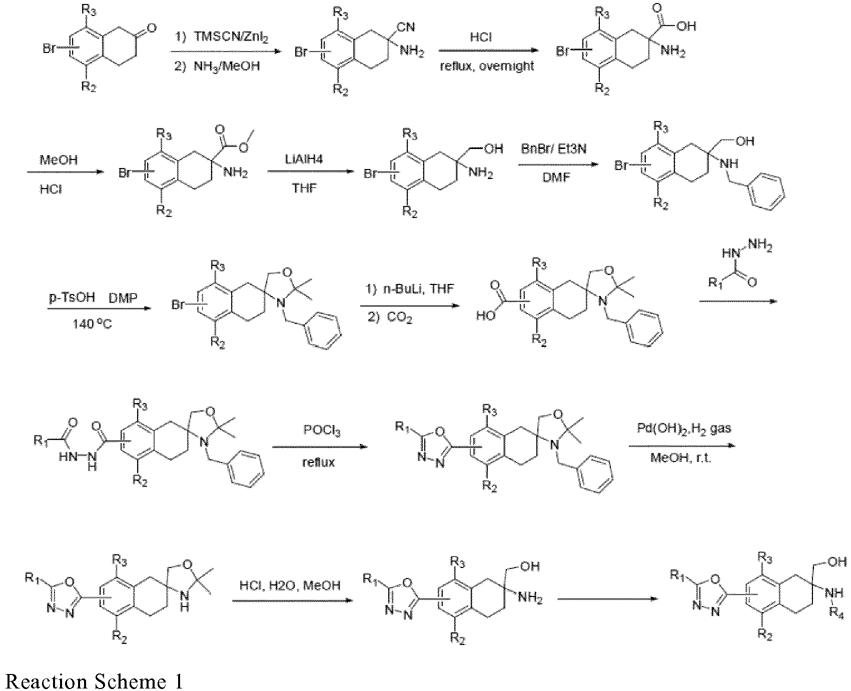

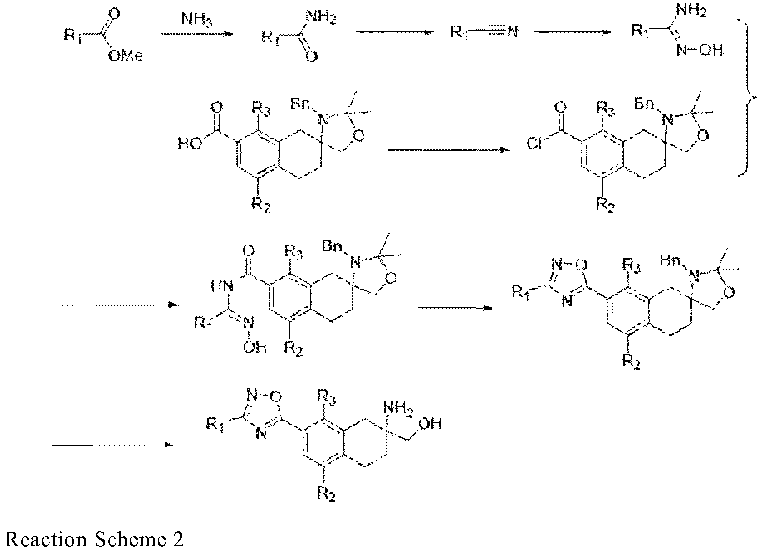

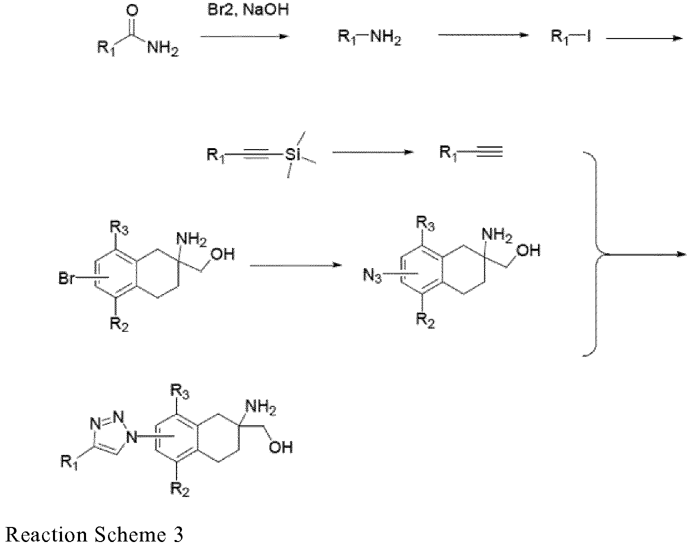

The present invention provides the compound of Formula I and the salt thereof for use as an immunosuppressive drug.

The present invention provides the compound of Formula I and the salt thereof for use in the treatment of treating transplant rejection.

The present invention provides the compound of Formula I and the salt thereof for use in the treatment of immune mediated inflammatory diseases (or immune inflammatory diseases).

The present invention provides the compound of Formula I and the salt thereof for use in the treatment of multiple sclerosis, systemic lupus erythematosus and rheumatoid arthritis.

The compound according to the invention can also exist in various polymorphic forms, such as amorphous and crystalline polymorphic forms. It is a further aspect of the present invention that all the polymorphic forms of the compound according to the invention are included in the scope of the present invention.

As described below, all the phrases "compound of the Formula (I)" refer to the compound of Formula (I), and the salts, solvates or physiologically functional derivatives thereof as described herein.

The amount of the compound of Formula (I) for reaching the desire biological effect depends on numerous factors, such as the specific compound, the specific use, administration regime and the condition of the patient. Generally, the daily dosage is from 20mg to the 200mg, e.g., from 20mg to 50mg. For the prevention or treatment of the above diseases, the compound of Formula (I) may be used alone, but a pharmaceutical composition containing the compound and some tolerable excipients are preferably. Excipients must be tolerable in the respects of being compatible with other components of the composition and harmless to the patient's health. The excipients may be solid or liquid, or both, preferably prepared in combination of the compound according to the invention into a single unit dose, such as a tablet, which may contain 0.05 to 95 weight percent of the compound. The composition may include other pharmaceutically active substances including other compounds according to Formula (I). The pharmaceutical composition according to the invention can be prepared through one of the known pharmaceutical preparation methods, which are essentially mixing the components and pharmaceutically acceptable excipients and/or auxiliaries.

The pharmaceutical composition of the invention are those which is suitable for oral, rectal, local, peroral (e.g. sublingual) and parenteral (such as subcutaneous, intramuscular, intradermal or intravenous) administration. However, the most suitable administration route for each specific case depends on the property and severity of the disease in each case and the properties of the compound of Formula (I). Sugar coated preparation and sugar coated delayed-release preparation are also included in the scope of the present invention. Acid resistant and enteric preparations are preferred. Suitable enteric coatings include cellulose acetate phthalate, polyethylene acetate phthalate, hydroxypropyl methyl cellulose phthalate and anionic polymer of methacrylic acid and methacrylate.

The pharmaceutical compositions suitable for oral administration may be an individual unit dosage, such as a capsule, a cachet, a plating or tablet, each of them containing certain amount of a compound of Formula (I); powder or granule; solution or suspension in aqueous or anhydrous liquid; emulsion of oil in water or water in oil. As mentioned above, the composition may prepared through any suitable pharmaceutical methods, comprising the step of contacting the active compound with excipients (can be composed of one or more other components). In general, the composition is prepared by uniformly mixing the active compound and the liquid and/or fine crushing solid excipients, and molding the product if necessary. Thus, for example, the tablet may be prepared by compression of the compound: if appropriate, in combination with one or more additional components powder or granules or make them molding. Compressed tablets can be prepared, in the appropriate machinery, by firstly mixing the free flow form (such as powder or granular form) of the compound, if appropriately, with adhesives, lubricants, inert diluents and/or a kind of (amounts of) surfactants/dispersants, and then pressing into tablets. Molded tablets can be prepared, in the appropriate machinery, by moulding the powder compounds wetting by inert liquid diluents.

The pharmaceutical compositions suitable for oral administration (sublingual) include lozenge, which contains the compound of Formula (I) and corrigent (e.g., sucrose and Arabic gum), and pastille, which contains the compound in an inert medium such as gelatin and glycerol or sucrose and Arabic gum.

The pharmaceutical compositions suitable for parenteral administration preferably include sterile aqueous preparation of the compound of Formula (I), which are preferably isotonic with the blood of subjects. The pharmaceutical compositions are preferably administered intravenously, and may also administered subcutaneously, intramuscularly or intradermally. These compositions can be preferably prepared, by mixing the compounds and water to obtain a solution, and then make the solution sterile and isotonic with blood. The injectable compositions according to the present invention generally comprise 0.1 to 5 % of active compounds in weight.

The pharmaceutical compositions suitable for rectal administration are preferably single dose suppositories. They may be prepared, by mixing compound of Formula (I) with one or more conventional solid excipients, such as cocoa butter, and molding the obtained mixture.

The pharmaceutical compositions suitable for topical administration are preferably ointment, cream, paste, spray, aerosol or oil. The available excipients are petroleum Jelly, lanolin, polyethylene glycol, alcohol and combinations of two or more of these above substances. The concentration of active compounds generally accounts for 0.1 to 15% of the weight of the composition.

Cutaneous penetration is feasible. The pharmaceutical compositions suitable for cutaneous penetration can be single patches, which are suitable for long close contact with the patient skin. This kind of patch relates to optional buffered aqueous solutions containing active compounds, and the compounds are dissolved and / or dispersed in adhesives or dispersed in polymers. The suitable concentration of active compounds is arranged from 1 % to 35 %, preferably from about 3 % to 15 %. It is especially possible that active compounds may be released through electron transportation or iontophoresis, e.g., as described in Pharmaceutical Research, 2 (6): 318 (1986).

The compound of the present invention and the physiological salts thereof only target sphingosine 1 phosphate receptor 1 (S1P1), down-regulate S1P1 expression, and inhibit lymphocytes into the peripheral blood circulation, which are useful for the treatment of immune mediated inflammatory diseases such as

| Instrument model | Shimadzu LCMS-2020 System. |
|---|---|
| HPLC column: | XBridge C18 3.5um 3.0×50mm. |
| Column temperature | 40 °C |
| PDA wavelength: | 254 nm. |
| LC Pump Total Flow: | 1 ml/min. |
| Pump A: | 0.05% Formic acid solution in water; |
| Pump B: | 0.05% Formic acid solution in acetonitrile |
| LC Time Program: | 0-2.1 min, Pump B 5-100%; |
| | 2.1-3.5 min, Pump B 100%; |
| | 3,51 min, Pump B 5%; |
| | 6.5 min, Stop. |
| MS API Unit: | ESI; |
| MS Scan m/z: | 100-800(Event 1, Event2). |
| Result | [M-H]⁻, [M+H]⁺ |

multiple sclerosis, rheumatoid arthritis, etc.

### Embodiments

### Experimental Parts:

### I. Synthesis of compounds:

1. The LCMS analysis conditions in the synthesis process are as shown below:
   LC Analysis

### 2. Synthesis

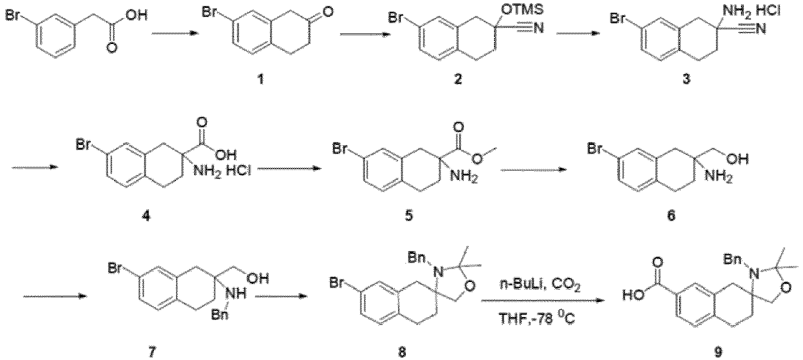

### Reaction Scheme 4: Preparation of Intermediate 9

### Preparation of Intermediate 1:

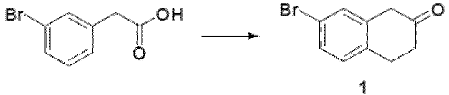

The raw material meta-bromophenylacetic acid (100 g, 0.47 mol, 1.0 eq) was dissolved in dichloromethane (500 ml), then was cooled to 0 °C in ice-salt bath. Oxalyl chloride (120 g, 0.95 mol, 2.0 eq) was added dropwise to the reaction mixture with temperature maintaining 0 °C. After the dropping was finished, the reaction temperature was changed to room temperature and reacted for two hours. The reaction mixture was concentrated under reduced pressure to obtain meta-bromophenylacetyl chloride (110 g, 0.47 mol) after the reaction completed. The obtained meta-bromophenylacetyl chloride was dissolved in dichloromethane (200 ml) for further use.

AlCl₃ (210 g, 1.9 mol, 4.0 eq) and dichloromethane (500 ml) were added to another reaction flask. The system was cooled to -5 °C in ice-salt bath, and was dropped into the solution of the prepared meta-bromophenylacetyl chloride in dichloromethane described above, maintaining the temperature of -5 °C, then ethylene gas was introduced into the system maintaining the temperature between -5 °C∼0 °C, with continuously three hours of ethylene gas. After completion of the reaction, the reaction mixture was poured into ice water (5000 ml), which was then extracted with dichloromethane (3 x 1000 ml). The organic layer were combined and washed once with water (1000 ml), dried over anhydrous Na₂SO₄, and filtered. The mother liquor was concentrated under reduced pressure to obtain a residue, which was then purified by column chromatography (ethyl acetate: heptane =1: 30) resulting in intermediate 1 (45 g, yield: 43 %) as a yellow solid. MS (M + H⁺): 225, 227, the theoretical calculating value: 225, 227.

### Preparation of Intermediate 3:

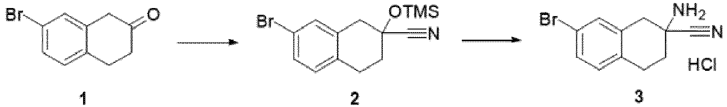

Under the protection of nitrogen, raw material of 1 (20.0 g, 89 mmol, 1.0 eq) and ZnI (1.4 g, cat.) were added to trimethyl silylcyanide (13.0 g, 130 mmol, 1.5 eq), and the reaction system reacted at room temperature for 3 hours to produce 2. NH₃/methanol solution (50 ml) was added to the reaction system, and then reacted at room temperature over night. After the reaction completed, the system was concentrated under the reduced pressure, and the resulting residue was dissolved in HCl/methanol (50 ml). Diethyl ether (200 ml) was added and large amounts of solid precipitated, then filtered, and the filter cake was dried to obtain intermediate compound 3 (23 g, 89 %) as a yellow solid. MS (M + H⁺): 251, the theoretical calculating value: 251.

### Preparation of Intermediate 4:

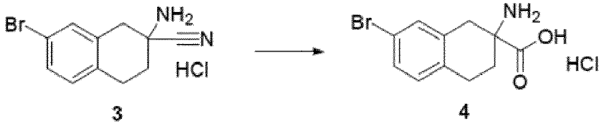

Intermediate 3 (23 g, 80 mmol, 1 eq) was added to 8N hydrochloric acid (200 ml), then was heated to reflux and reacted over night. After the completion of the reaction, the reaction solution was cooled to room temperature, then filtered, and the filter cake was collected and dried to obtain intermediate compound 4 (20 g, 81 %) as a yellow solid. MS (M + H⁺): 271, the theoretical calculating value: 271

### Preparation of Intermediate 5:

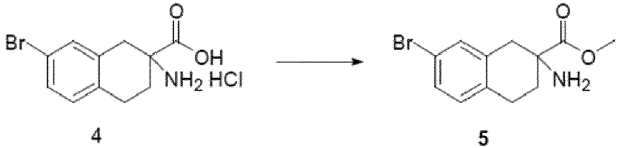

Raw material 4 (20.0 g, 65 mmol, 1.0 eq) was added to the methanol (500 ml), then cooled to 0 °C. Thionyl Chloride (23 g, 195 mmol, 3.0 eq) was dropped in the reaction system which was then heated to reflux and reacted for 3 hours. After the reaction completed, the system was concentrated under reduced pressure. Water (300 ml) was added to the resulting residue and saturated sodium bicarbonate was used to regulate the pH (pH=8). The aqueous layer was extracted three times by ethyl acetate (200 ml), and organic layer was combined and washed by aqueous sodium chloride one time, then dried over anhydrous sodium sulfate, and filtered. The mother liquor was concentrated under reduced pressure to give intermediate compound 5 (15.0 g, 81 %) as black oil. MS (M + H⁺): 285, the theoretical calculating value: 285.

### Preparation of Intermediate 6:

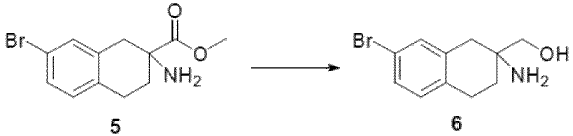

LiAlH₄ (4.0 g, 106 mmol, 2.0 eq) was added to tetrahydrofuran (200 ml), and the system was cooled to 0 °C. Solution of raw material 5 (15.0 g, 53 mmol, 1.0 eq) in tetrahydrofuran (100 ml) was dropped in, then reacted at 0 °C for 30 minutes. After the completion of the reaction, water (4 ml), and sodium hydroxide (8 ml) were added to the system, and then the mixture was filtered. The mother liquor was concentrated under reduced pressure to obtain intermediate compound 6 (12.0 g, 88 %) as black oil. MS (M + H⁺): 256, the theoretical calculating value: 256.

### Preparation of Intermediate 7:

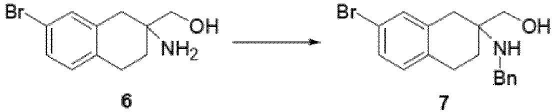

Raw material 6 (13.0 g, 51 mmol, 1.0 eq), acetic acid (4.5 g, 150 mmol, 1.5 eq) and benzaldehyde (5.3 g, 51 mmol, 1.0 eq) were added to dichloromethane (200 ml), and cooled to 0 °C. Sodium borohydride acetate (16.0 g, 150 mmol 1.5 eq) was added portionwise, then reacted at 25 °C for 30 minutes. After the completion of the reaction, sodium hydroxide (8 ml) and saturated sodium bicarbonate (100 ml) were added to the system, which was then extracted with dichloromethane (100 ml) for three times. The organic layer was dried over anhydrous sodium sulfate, filtered, and concentrated under reduced pressure to give a crude product, which was purified by silica gel column chromatography (ethyl acetate: heptane = 1: 10) to obtain intermediate compound 7 (13.0 g, 74 %) as brown oil. MS (M + H⁺): 346, the theoretical calculating value: 346.

### Preparation of Intermediate 8:

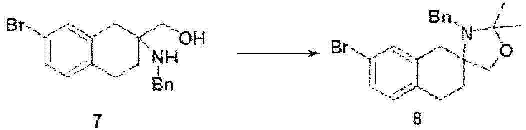

Raw material 7 (13.0 g, 38 mmol, 1.0 eq) and TsOH (0.3 g, cat.) were added to 2, 2-dimethoxypropane (100 ml), heated to 120 °C and reacted over night. After the completion of the reaction, the system was concentrated under reduced pressure. Water (100 ml) and saturated sodium bicarbonate (50 ml) were added to the resulting residue, which was then extracted with dichloromethane (100 ml) for three times. The organic layer was dried over anhydrous sodium sulfate, filtered, and concentrated under reduced pressure to give a crude product, which was purified by column chromatography (ethyl acetate: heptane = 1: 20) to obtain intermediate compound 8 (9.4 g, 65 %) as a pale yellow solid. MS (M + H⁺): 386, 388, the theoretical calculating value: 386, 388.

### Preparation of Intermediate 9:

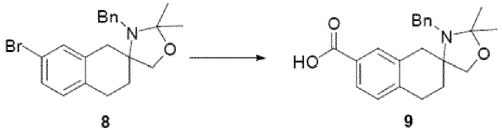

Raw material 8 (5.0 g, 13 mmol, 1.0 eq) was added to tetrahydrofuran (50 ml), and cooled to -78 °C under nitrogen atmosphere. N-BuLi (7.8 ml, 1.5 eq) was added dropwise to the system and the system was reacted at -78 °C for 30 minutes, then dry ice (2 g) was added. After that, the temperature was raised naturally to the room temperature. The system was concentrated under reduced pressure, and water (50 ml) was added to the resulting residue. The pH value was regulated to 4 by 2N HCl, then filtered, and the filter cake was collected and dried to give intermediate compound 9 (4.0 g, 88 %) as a white solid. MS (M + H⁺): 352, the theoretical calculating value: 352.

### Preparation of Intermediate 18:

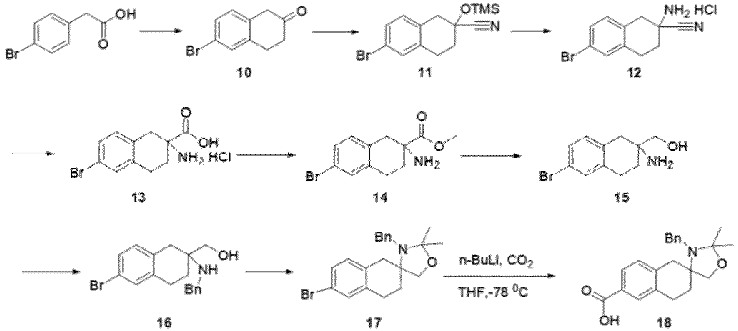

The Reaction Scheme 5: Preparation of Intermediate 18

Intermediate 18 was obtained through Scheme 4 with reference to the synthesis method of Intermediate 9.

### The synthesis of Example 1:

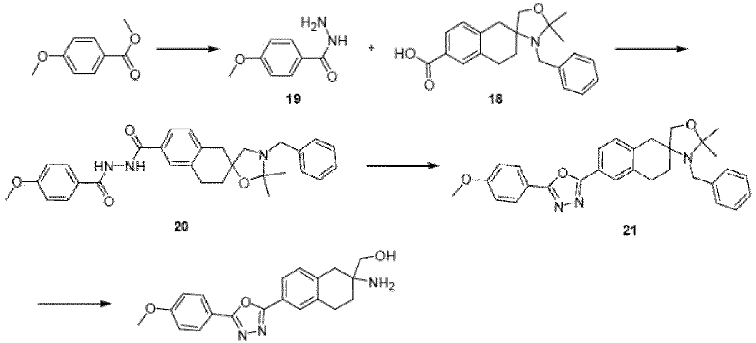

The Reaction Scheme 6: The Synthesis of the compound in Example 1

### Preparation of Intermediate 19:

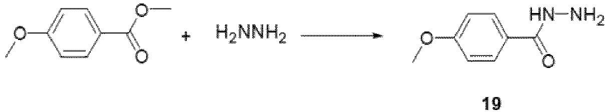

The mixture of 4-methoxy methyl benzoate (33 g, 0.2 mol) and anhydrous hydrazine (7.7 g, 0.24 mmol) was heated to 140 °C under nitrogen atmosphere, and reacted at the said temperature for 30 minutes. After cooled to the room temperature, the mixture was extracted with ethyl acetate (3×100 ml). The organic layer was dried over anhydrous sodium sulfate, and filtered, then concentrated under reduced pressure to give a crude product of intermediate compound 19 (30 g), which can be used for the next reaction step directly.

### Preparation of Intermediate 20:

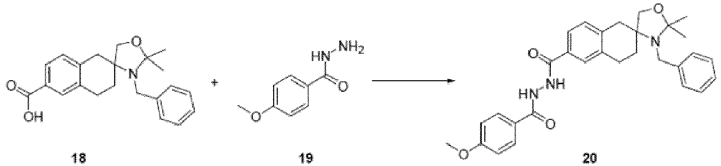

Raw material 18 (0.5 g, 1.0 eq) was dissolved in the dried dichloromethane (20 ml), and DMF (0.01 g, cat.) was added, then the temperature of the system was lowered to 0 °C. Oxalyl chloride (500 mg, 3.0 eq) was added dropwise to the system, after which the temperature was raised to room temperature naturally and reacted for 30 minutes. After the completion of the reaction, the system was concentrated under reduced pressure to obtain acyl chloride which was dissolved in dichloromethane (10 ml) for further use.

Compound 19 (240 mg, 1.0 eq) and dichloromethane (20 ml) were added to another reaction flask, and Et₃N (430 mg, 3.0 eq) was added at room temperature, and then cooled to 0 °C. The prepared solution of acyl chloride in dichloromethane described above was added dropwise to the system, then heated to room temperature naturally and reacted for 1 h. The reaction was completed under the monitoring of LCMS and Thin-Layer Chromotography (ethyl acetate: petroleum ether=1: 1). Water (30 ml) was added to the reaction solution which was then extracted with dichloromethane (3×30 ml). The organic layer was dried and concentrated to give a crude product, which was purified by column chromatography (ethyl acetate: petroleum ether = 1: 5) to obtain intermediate compound 20 (200 mg).

### Preparation of Intermediate 21:

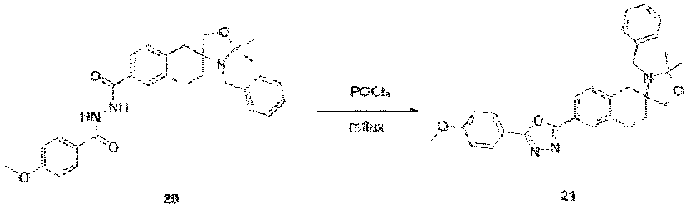

Raw material 20 (100 mg, 1.0 eq) was dissolved in POCl₃ (10 ml), then heated to influx, and reacted for 3 hours. The reaction was completed under the monitoring of LCMS and Thin-Layer Chromotography (dichloromethane: methanol=10: 1). The reaction solution was concentrated under reduced pressure to get rid of POCl₃. Ice water (20 g) was added to the resulting residue, and sodium bicarbonate was added to regulate the PH to 8, which was then extracted with dichloromethane (3×30 ml). The organic layer was dried and purified by column chromatography (dichloromethane: methanol=30: 1) to obtain intermediate compound 21 (50 mg).

### Example 1: 6-[5-(4-methoxyphenyl)-2-[1,3,4]oxdiazolyl]-2-amino-1,2,3,4-tetrahydro-naphthalen-2-yl-methanol

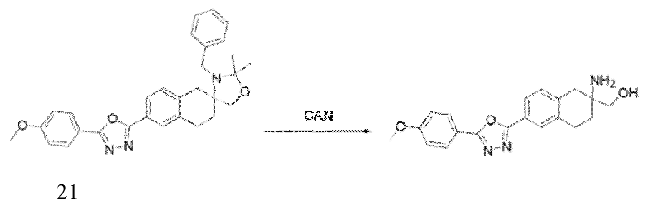

### Example 1

Raw material 21 (45 mg, 0.094 mmol, 1.0 eq) and ammonium ceric nitrate (45 mg) were added to the mixture of acetonitrile (10 ml) and dichloromethane (1 ml). The system was reacted for 5 hours at room temperature, which was under the monitoring of Thin-Layer Chromotography (dichloromethane: methanol=10: 1) and LCMS. After the completion of the reaction, water (20 ml) was added to the system, which was then extracted with dichloromethane (3×20 ml). The organic layer was combined and washed with saturated aqueous sodium chloride solution (20 ml), dried over anhydrous sodium sulfate, filtered, and concentrated under reduced pressure to give a crude product, which was purified by column chromatography (dichloromethane: methanol=50: 1) to obtain the compound in Example 1 (20 mg, 61 %) as a white solid. NMR (400 MHz, CDCl₃) δ = 7.42 (2H, d), 7.32 (2H, d), 7.21 (2H, m), 6.90 (1H, d), 3.86 (3H, s), 3.70 (2H, dd), 2.80 (4H, m), 1.82 (2H, m); Rt = 3.12 min, MS (M + H⁺): 352, the theoretical calculating value: 352.

The following examples were obtained by synthesis method similar as that used in Example 1:

### Example 20: 7-[5-(3, 4-diethoxyphenyl)-2-[1, 3, 4]-oxdiazolyl]-2-amino-1, 2, 3, 4-tetrahydro-naphthalen-2-yl-methanol

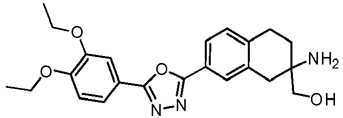

LC-MS: 410.2 [M+1]⁺, t_{R}=1.822 min. ¹H NMR (400 MHz, DMSO) δ 7.90 - 7.77 (m, 2H), 7.68 (dd, *J* = 8.4, 1.9 Hz, 1H), 7.59 (d, *J* = 1.9 Hz, 1H), 7.33 (d, *J* = 7.9 Hz*,* 1H), 7.17 (d, *J* = 8.5 Hz, 1H), 4.98 (br s, 1H), 4.14 (p, *J* = 6.8 Hz, 4H), 3.29 (s, 2H), 3.04 - 2.60 (m, 4H), 1.83 - 1.59 (m, 2H), 1.41 - 1.33 (m, 6H).

### 1. S₁P₁-GTP γ S binding assay

### Experimental Materials

1) CHO-S₁P₁ cell lines
2) T-75 cell culture plate; 150 mm cell culture dish
3) DMEM cell culture fluid; 10 % (v/v) fetal bovine serum; 100 U/ml penicillin-100 µg/ ml streptomycin; 2 mM L-oryzanol, 1 mg/ml G-418
4) Cell Lysis Buffer: HEPES20 mM (pH 7.4), EDTA 10 mM
5) Stock solution: HEPES 20 mM, EDTA 0. 1 mM
6) Binding Buffer: HEPES 20 mM (pH 7.4), NaCl 100 mM, MgCl₂ 5 mM, GDP 1 µM, BSA 0.1 % (w/v), DMSO 2.5 % (v/v),
7) Washing solution: NaH₂PO₄ 10 mM (pH 7.4)

### Equipment

Super clean bench for cell culture
Cell Crushing machine
Beckman centrifugal machine
Microporous (MicroBeta) 96-well collector
2450 MicroBeta ²_{TM} Liquid scintillation counter/detector

### Test Method

The test compounds were dissolved in DMSO and prepared as a concentration of 20 mM.

The concentrations of the compounds were detected ranging from 0.3 nM to 20 µM.

Control group: 10 M S1P and SEW2871 were used as positive control and were purchased from Tocris company, and the binding buffer was as negative control.

Test method: wetting membranes by saponin solution, and preparing [³⁵S]-GTP γ S in binding solution (4X). The test compound was prepared as 4X of the final concentration which was then performed serial 3X dilution in 100 % DMSO. Membranes with high-expressed S1P1 were collected in 5 ml binding buffer, and 50 µL was added to 96-well (containing 5µg membrane protein with high-expressed S1P1). 25 µL test compound was added in each well. The compound was incubated with the membranes for 30 min at room temperature, then 25 µL [³⁵S]-GTP γ S was added, and the experiment was initiated. The 96-well detection plate was incubated for 1 hour at room temperature, filtered by filtermat B, and washed with washing solution for three times. After the plate was dried for 1 h at 50°C, 8 ml scintillator solution was added and counted using scintillation counter.

Result Analysis and Determination of 50 % inhibitory concentration (IC₅₀):
The IC₅₀ value was calculated by using (Prism 4) software, and 50% inhibitory concentrations were obtained.

**Table 1. Representative compounds and activities:**

| Examples | Name | IC₅₀ (nM) |
|---|---|---|
| 20 | 7-[5-(3, 4-diethoxyphenyl)-2-[1, 3, 4]-oxdiazolyl]-2-amino-1, 2, 3, 4 -tetrahydro- naphthalen-2-yl-methanol | 179 |

### 2. S1P1 β-arrestin Assay

### 2.1 Experimental Materials: Path hunter express CHO-K1 EDG1 β-arrestin GPCR Assay was purchased from DiscoveRx, containing CHO-EDG-1 cells, buffers, substrate-1, substrate-2, cell culture fluid, 96-well cell culture plate.

### 2.2 Equipment

1) Super-clean bench for cell culture
2) Evision 2104 cell plate counter
3) 37 °C incubator

### 2.3 Test Method

1) The test compounds were dissolved in DMSO and prepared as a concentration of 20 mM.
2) The concentrations of the test compounds were detected ranging from 0.3 nM to 20 µM.
3) Control group: 0.045nM-3µM S1P purchased from Tocris company; while the binding buffer was used as the negative control.
4) Assay procedures: OCC culture solution was preheated in water tank at 37 °C. Cells were taken out from liquor nitrogen tank, left on the dry-ice, and dissolved in water tank at 37°C. 0.5ml OCC culture solution was added and blended slowly. The cells were diluted in 11.5mL OCC culture solution, and 100 µL cell suspension (containing 8333 cells) was added to each well of the 96-well plate. The 96-well plate was incubated for 8 hours under 5% CO₂ at 37 °C, and 10 µL test compound dissolved in DMSO was added in; 10 µL DMSO was used as negative control. After the plate was incubated for 90 min at 37°C, 55 µL detection solution was added to each well. Then the results were read out by EnVision after the cell plate was incubated for 90 min at room temperature.

### 3. Effects of the compound on Peripheral Blood Lymphocyte in Mice

### 3.1 Experimental Materials:

1) 15 % EDTA, PBS, EP tubes, 15 ml centrifuge tube, capillary tube, pipette tip were all purchased from Shanghai biological technology co., Ltd. Hemocyte analysis reagents were purchased from Shanghai Sysmex Company.
2) 35 Balb/c mice (female, 20-22g), supplied by laboratory animal center of Jilin University.
3) Automated Hematology Analyzer (pocH-100iV Diff) was purchased from Sysmex Company, Kobe, Japan.

### 3.2 Assay Method:

1) The stock solutions of the compound were prepared with normal saline, and the concentrations are 2 mg/ml and 20 mg/ml, respectively.
2) The mice were divided into 5 groups, i.e., control group, low-dose experimental group, and high-dose experimental group. They were weighed and labeled well.
3) Before administration, supraorbital venous blood was collected with 20 µl /mouse which was added to anticoagulant tube containing EDTA, and mixed thoroughly. The numbers of lymphocytes were analyzed by pocH-100.
4) The control group was given normal saline; low-dose experimental groups were given the compound (5 mg/kg/d); high-dose experimental groups were given the compound (30 mg/kg/d), with liquid amount of 500 µl /mouse, by intragastric administration. Administration was performed for continuous four days, once a day. After 3.5 hours of the first and fourth administration, blood was collected respectively, and changes in the numbers of lymphocytes were analyzed by pocH-100.

### 4. Experimental Autoimmune Multiple Sclerosis

### 4.1 Materials

1) SJL/J mice (8-9 weeks, female) were purchased from JAX Lab.
2) PLP139-151 (HSLGKWLGHPDKF amide) was ordered from Biosource Internaional Company (QCB).
3) Incomplete Freund's adjuvant (IFA) was purchased from Difco Company.
4) Mycobacterium tuberculosis H37 was purchased from Difco Company.
5) Escherichia coli pertussis toxin was purchased from Calbiochem Company.
6) 1.0 ml insulin injection syringes, 10 ml injectors, tee connectors.

### 4.2 Immunization of mice

1) Preparation of Complete Freund's adjuvant (CFA): Mycobacterium tuberculosis H37 was added to incomplete freund's adjuvant (IFA) to produce complete freund's adjuvant (CFA) containing 8.0 mg / ml mycobacterium tuberculosis.
2) PLP polypeptides were dissolved in PBS solution to prepare 10 mg/ml, and the working concentration is 2.0 mg/ml.
3) PLP polypeptide antigens were mixed with CFA.
4) Immunizing mice: each mouse was inoculated with 100 µg PLP139-151 and 0.4 mg mycobacterium tuberculosis.
5) At the day of immunization and the next day, each mouse was intraperitoneally injected with 100 ng pertussis toxin.

### 4.3 Assay Method:

1) The stock solutions of the compound were prepared with 20% 2-hydroxypropyl-β-cyclodextrin into a concentration of 2 mg/ml and 20 mg/ml, respectively.
2) The mice were divided into 5 groups, i.e., control group, low-dose experimental group, and high-dose experimental group. They were weighed and labeled well.
3) The Control group was treated with 20% 2-hydroxypropyl-β-cyclodextrin; low-dose experimental group was given the compound (5 mg/kg/d); high-dose experimental group was given the compound (30 mg/kg/d), with liquid amount of 500 µl /mouse, intragastric administration. Administration was performed at the next day after immunization and continuously for 30 days, once a day. The changes of the animals' diseases were recorded every day.

### 4.3 The standard for evaluation scores of the diseases: 0, normal; 1, tail paralysis; 2, hind limbs weakness; 3, hind limbs paralysis; 4, fore and hind limbs paralysis; 5, impending death or death.

### 5. Experimental Rheumatoid Arthritis

### 5.1 Material

1) 10 mM acetic acid, 0.2 µm filter;
2) Incomplete Freund's Adjuvant (IFA), mycobacterium tuberculosis (inactivated strain H37Ra), were purchased from Difco Company.
3) DBA/1JLacJ mice (Jackson Lab)
4) Bovine collagen type II was purchased from Chondrex Company.

### 5.2 Preparation of antigens

1) Bovine collagen type II was dissolved in 10 mM acetic acid, and stayed overnight at 4°C.
2) The heat-inactivated mycobacterium tuberculosis was ground by mortar and pestle, and was prepared into complete Freund's adjuvant (CFA) at final concentration of 4 mg/ml.

### 5.3 Experiment Method:

1) The stock solutions of the compound were prepared with 20% 2-hydroxypropyl-β-cyclodextrin into a concentration of 2 mg/ml and 20 mg/ml, respectively.
2) The mice were divided into 5 groups, i.e., control group, low-dose experimental group, and high-dose experimental group. They were weighed and labeled well.
3) The Control group was treated with 20% 2-hydroxypropyl-β-cyclodextrin; low-dose experimental group was given the compound (5 mg/kg/d); high-dose experimental group was given the compound (30 mg/kg/d), with liquid amount of 500 µl /mouse, intragastric administration. Administration was performed at the next day after immunization and continuously for 30 days, once a day. The changes of the animals' diseases were recorded every day.

5.4 The development and evaluation on arthritis of the immunized mice: each mouse was injected with the mixture of 100 µg mycobacterium tuberculosis and 100 µg bovine collagen type II.

5.5 Evaluation on the development of arthritis: arthritis was usually occurred after 3∼5 weeks, and reached peak in the 6th week.

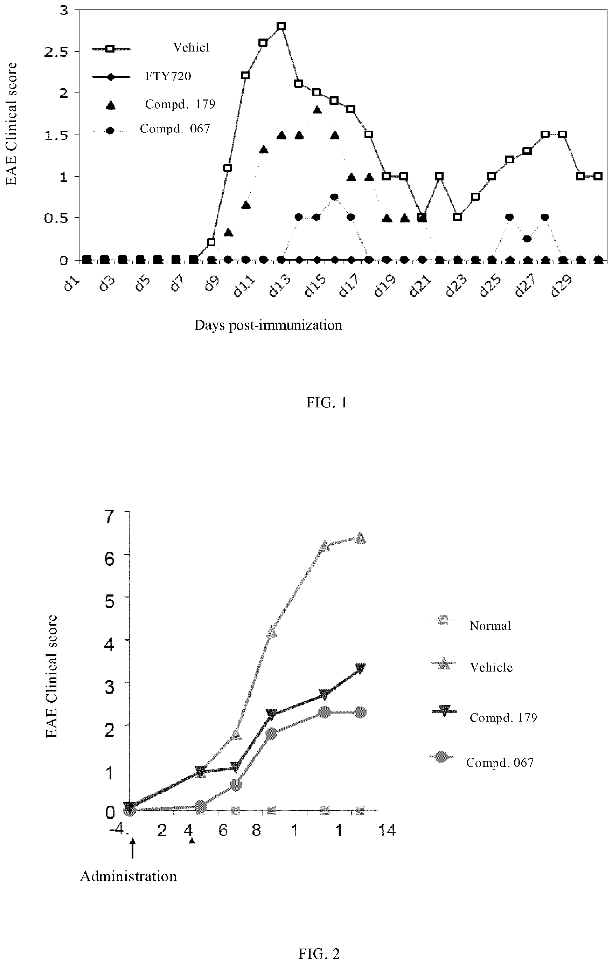

## Claims

1. A heterocyclic amino-methanol compound which is 7-[5-(3, 4-diethoxyphenyl)-2-[1, 3, 4]-oxdiazolyl]-2-amino-1, 2, 3, 4-tetrahydro-naphthalen-2-yl-methanol
and a physiologically acceptable salt thereof.

2. A compound according to claim 1 and the salt thereof for use in inhibiting the immune system or for use in the treatment of organ transplant rejection.

3. A compound according to claim 1 and the salt thereof for use in the treatment of immune mediated inflammatory diseases.

4. The compound for use according to claim 3 and the salt thereof, wherein the diseases are selected from the group consisting of multiple sclerosis, systemic lupus erythematosus and rheumatoid arthritis.

## Patentansprüche

1. Heterozyklische Aminomethanol-Verbindung, die 7-[5-(3,4-Diethoxyphenyl)-2-[1,3,4]-oxdiazolyl]-2-amino-1,2,3,4-tetrahydro-naphthalen-2-yl-methanol ist, und ein physiologisch verträgliches Salz davon.

2. Verbindung nach Anspruch 1 und das Salz davon zur Verwendung in der Inhibierung des Immunsystems oder zur Verwendung in der Behandlung von Organtransplantatabstoßung.

3. Verbindung nach Anspruch 1 und das Salz davon zur Verwendung bei der Behandlung von immunvermittelten entzündlichen Erkrankungen.

4. Verbindung zur Verwendung nach Anspruch 3 und das Salz davon, wobei die Erkrankungen ausgewählt sind aus der Gruppe, bestehend aus Multipler Sklerose, systemischem Lupus erythematodes und rheumatoider Arthritis.

## Revendications

1. Composé amino-méthanolique hétérocyclique qui est le 7-[5-(3,4-diéthoxyphényl)-2-[1,3,4]-oxdiazolyl]-2-amino-1,2,3,4-tétrahydro-naphtalén-2-yl-méthanol et un sel physiologiquement acceptable de celui-ci.

2. Composé selon la revendication 1 et sel de celui-ci destinés à être utilisés dans l'inhibition du système immunitaire ou destinés à être utilisés dans le traitement d'un rejet de greffe d'organe.

3. Composé selon la revendication 1 et sel de celui-ci destinés à être utilisés dans le traitement de maladies inflammatoires à médiation immunitaire.

4. Composé selon la revendication 1 et sel de celui-ci, dans lesquels les maladies sont sélectionnées dans le groupe constitué par la sclérose en plaques, le lupus érythémateux disséminé et la polyarthrite rhumatoïde.
